# FASCICULE DE BREVET EUROPEEN

(11) **EP 1 095 937 B1**
(45) Date de publication et mention de la délivrance du brevet: **12.03.2003**
(21) Numéro de dépôt: 00402697.7
(22) Date de dépôt: 29.09.2000
(51) Int. Cl.: C07D 263/32, C07D 413/04

(54) **Procédé de préparation d'oxazoles trisubstitués**
Verfahren zur Herstellung von trisubstituierten Oxazolen
Process for the preparation of trisubstituted oxazoles

(30) Priorité: 27.10.1999 FR 9913446
(43) Date de publication de la demande: 02.05.2001
(73) Titulaire: L'OREAL, 75008 Paris (FR)
(72) Inventeur: Dalko, Maria, 91190 GIF S/Yvette (FR); Dumats, Jacqueline, 93420 Villepinte (FR)
(74) Mandataire: Dodin, Catherine

(56) Documents cités:
- GB-A- 1 206 403
- SIEGRIST A E: "Über eine neue Synthese zur Darstellung heterocyclisch substituierter Stilbenverbindungen, die Anil-Synthese" HELVETICA CHIMICA ACTA, vol. 50, no. 3, 20 avril 1967 (1967-04-20), pages 906-57, XP002141560

## Description

La présente invention a trait à un nouveau procédé de synthèse d'oxazoles notamment trisubstitués.

Les oxazoles sont bien connus dans la littérature, qui en décrit plusieurs voies de synthèse possibles.
Ainsi, il est connu de les préparer par réaction d'un diazide sur un dérivé cyané, en présence de tungstène comme catalyseur (Tetrahédron Letters, 1974, 16, 1531). Ceci peut être illustré par le schéma suivant :

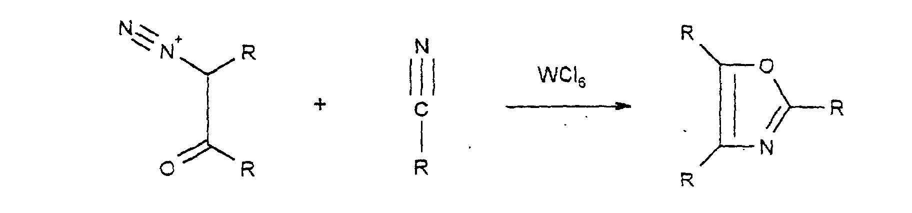

Il est également connu de préparer des oxazoles trisubstitués en utilisant une amine primaire comme réactif de cyclisation [J.A.C.S., 1943, 65, 2159], selon le schéma suivant :

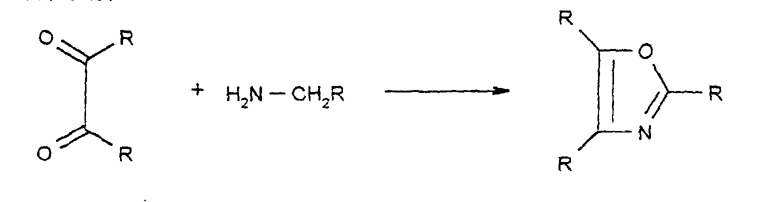

Toutefois, la synthèse des oxazoles trisubstitués selon ces deux procédés est liée à l'existence des composés de départ; or certains de ces produits de départ sont soit non disponibles commercialement, soit très difficilement synthétisables. En conséquence, selon la nature des substituants, certains oxazoles trisubstitués ne peuvent pas être préparés selon l'un ou l'autre de ces procédés.

On peut par ailleurs citer un procédé mettant en oeuvre la thermolyse et photolyse d'un dérivé d'acylisooxalone (J. Chem. Soc. Perkin, 1997, 2665), selon le schéma suivant :

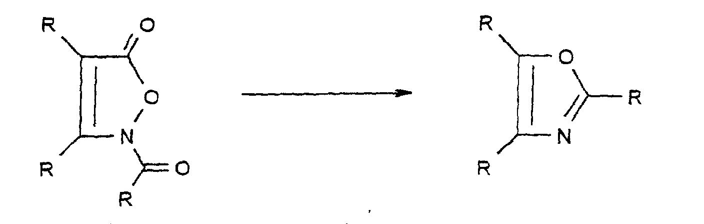

Toutefois, ce procédé requiert un appareillage bien particulier, permettant la photolyse des produits de départ, et n'est donc pas aisément industrialisable.

Il est encore connu, par US5719163, de procéder à une telle synthèse en utilisant l'acétate d'ammonium dans l'acide acétique à reflux, selon le schéma suivant:

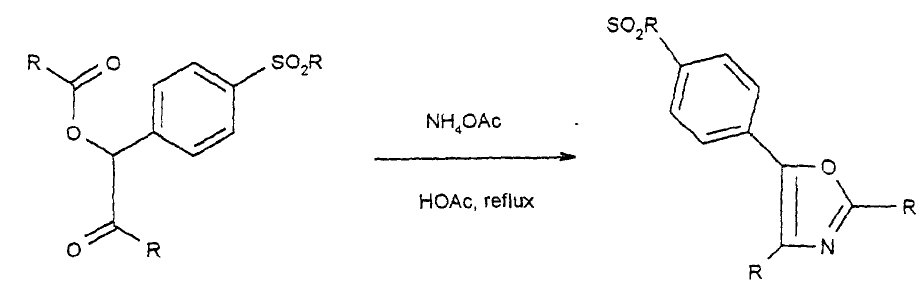

Ce procédé présente toutefois l'inconvénient d'être effectué en présence d'acide acétique, donc en milieu acide.

La présente invention a pour but de proposer un nouveau procédé de préparation d'oxazoles trisubstitués, pouvant être effectué à partir de produits de départ facilement synthétisables ou déjà disponibles commercialement, ne nécessitant pas d'appareillage spécifique et pouvant être effectué dans des conditions expérimentales, de pH notamment, plus favorables industriellement.

La présente invention a donc pour objet un procédé de préparation d'oxazoles trisubstitués de formule (II) : 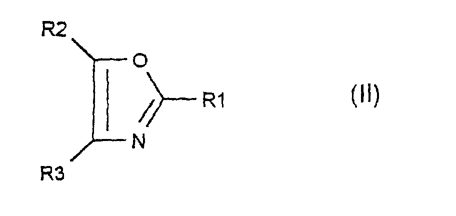 par réaction de thiourée sur la dicétone-ester correspondante de formule (I) : 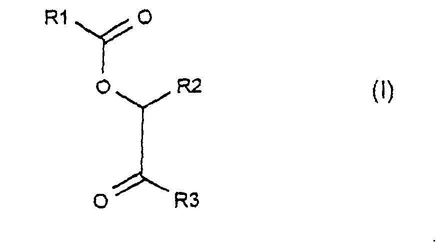 dans lesquelles les radicaux R₁, R₂ et R₃, identiques ou différents, ont les significations données ci-après.

On a constaté que le procédé selon l'invention pouvait être conduit à une valeur de pH basique, voire proche de la neutralité. Par ailleurs, les produits de départ sont aisément synthétisables, et de manière industrielle et peu onéreuse. De plus, la réaction mise en oeuvre ne comporte qu'une seule étape, ce qui peut également faciliter son industrialisation. Enfin, il est possible, grâce à ce procédé, de préparer une grande variété d'oxazoles trisubstitués.

La présente invention concerne donc un nouveau procédé de préparation d'oxazoles pouvant être trisubstitués, par réaction de thiourée sur la dicétone-ester correspondante, selon le schéma réactionnel suivant :

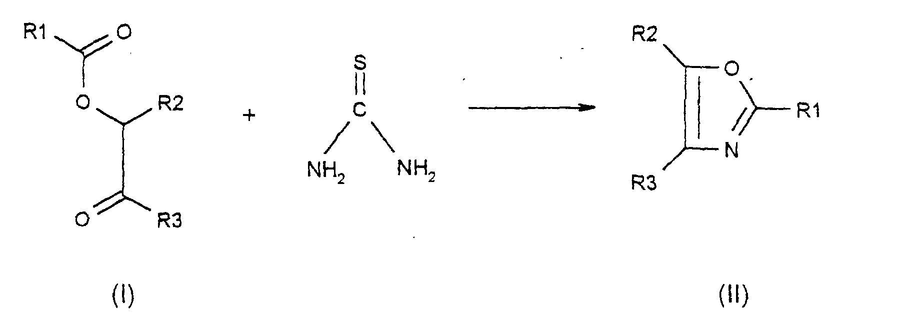

Dans le schéma réactionnel ci-dessus, les radicaux R₁, R₂ et R₃, identiques ou différents, peuvent être choisis parmi :
- les radicaux hydrocarbonés linéaires ou ramifiés, saturés ou insaturés, en C₁-C₁₂, éventuellement substitués par -OR, -SR, -NRR', -COOR, -CN, -SOₙCH₃, -CF₃ et/ou halogène (par exemple chlore ou fluor), avec R et R', identiques ou différents, représentant l'hydrogène, un radical hydrocarboné linéaire, ramifié ou cyclique, saturé ou insaturé (alkyl, aryl, aralkyl ou alkylaryl) en C₁-C₁₂ et n = 0, 1 ou 2;
- les radicaux aryles éventuellement substitués par un radical hydrocarboné linéaire ou ramifié, saturé ou insaturé, en C₁-C₁₂, un radical -OR, -NRR', -COOR, -CN, -SOₙCH₃, -SR, -CF₃ et/ou halogène (par exemple Cl, F), R, R' et n ayant les mêmes significations que ci-dessus, et/ou
- les radicaux hétérocycliques en C₅₋₁₀, saturés ou insaturés, comportant au moins un hétéroatome choisi parmi N, S et/ou O, éventuellement substitués par un radical hydrocarboné linéaire ou ramifié en C₁-C₁₂, un radical -OR, -NRR', -COOR, -CN, -SOₙCH₃, -SR, -CF₃ et/ou halogène (par exemple Cl, F), R, R' et n ayant les mêmes significations que ci-dessus.

De préférence, les radicaux R₁, R₂ et R₃, identiques ou différents, sont choisis parmi:
- les radicaux hydrocarbonés saturés, linéaires ou ramifiés, en C₁-C₆, notamment les radicaux méthyle, éthyle, propyle, n-butyle, iso-butyle ou ter-butyle;
- les radicaux hydrocarbonés linéaires ou ramifiés, saturés, en C₂-C₆ substitués par -OR avec R représentant un radical phényl ou alkylaryl, notamment un radical -(CH₂)ₙ-phényl avec n = 1 à 4, notamment 1 ou 2;
- le radical phényle;
- les radicaux phényles substitués par un radical hydrocarboné saturé en C₁-C₄, notamment méthyle, éthyle, propyle, n-butyle, iso-butyle ou ter-butyle;
- les radicaux phényles substitués par un radical -NRR' avec R et R' représentant un radical hydrocarboné saturé en C₁-C₄, notamment méthyle, éthyle, propyle, n-butyle, iso-butyle ou ter-butyle;
- les radicaux phényles substitués par au moins un halogène, notamment au moins un fluor;
- les radicaux hétérocycliques en C₅₋₆, insaturés, comportant au moins un atome d'azote dans le cycle.

La réaction peut notamment être effectuée dans un solvant dipolaire aprotique, ayant de préférence un point d'ébullition supérieur à 130°C, notamment supérieur à 140°C, et encore mieux supérieur à 160°C, par exemple dans le DMF (diméthylformamide).
La réaction est de préférence effectuée à une température d'au moins 130°C, notamment supérieure à 140°C, et encore mieux supérieure à 160°C, et encore plus préférentiellement à reflux du solvant.
La réaction peut également être effectuée en l'absence de solvant, sous vide, à une température proche ou supérieure du point de fusion d'un des deux réactifs.

D'une manière générale mais non limitative, le procédé selon la présente invention peut être mis en oeuvre de la manière suivante :
- on solubilise la dicétone-ester (I) et la thiourée dans le solvant, de préférence à raison de environ 2 équivalents de thiourée pour 1 équivalent de dicétone-ester; et
- on chauffe le mélange à une température d'au moins 130°C, pendant le temps nécessaire à l'accomplissement de la totalité de la réaction, et qui peut être de l'ordre de 5 à 10 heures.

Le produit recherché peut ensuite être isolé selon les méthodes usuelles, telles que précipitation, filtration, extraction.
Le produit recherché peut enfin être lavé et/ou séché et/ou recristallisé et/ou purifié selon les méthodes usuelles, si besoin est.
On obtient généralement le produit recherché avec un bon rendement.

L'invention est illustrée plus en détail dans les exemples suivants.

### Exemple 1 : Préparation du 2,4,5-triphényl-oxazole

### Schéma réactionnel :

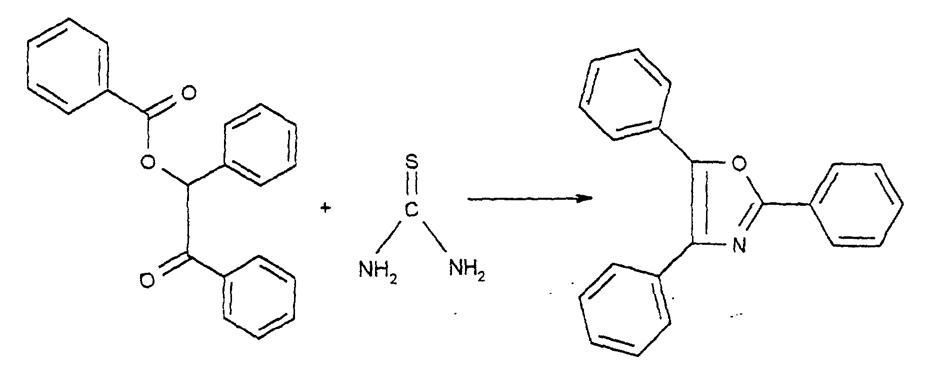

Dans un réacteur, on additionne 1 g de benzoate de désyle et 0,5 g de thiourée (2 éq.) dans 10 ml de diméthylformamide. La solution est laissée sous agitation à 160°C pendant 7 heures.
On verse la solution sur un mélange eau/glace. Le produit précipité est lavé à l'eau, filtré sur fritté sous vide et placé une nuit au dessiccateur avec P₂O₅
On purifie sur gel de silice (éluant : dichlorométhane) et l'on obtient 0,62 g d'une poudre blanche (rendement 68%).
- spectre RMN¹H (200MHz; CDCl₃)

| δ(ppm) | multiplicité | intégration |
|---|---|---|
| de 7,27 à 7,35 | m | 6 |
| 7,39 | m | 3 |
| 7,61 | m | 4 |
| 8,05 | m | 2 |

### Exemple 2 : Préparation du 2-méthyl-4-5 diphényl-oxazole

### Schéma réactionnel :

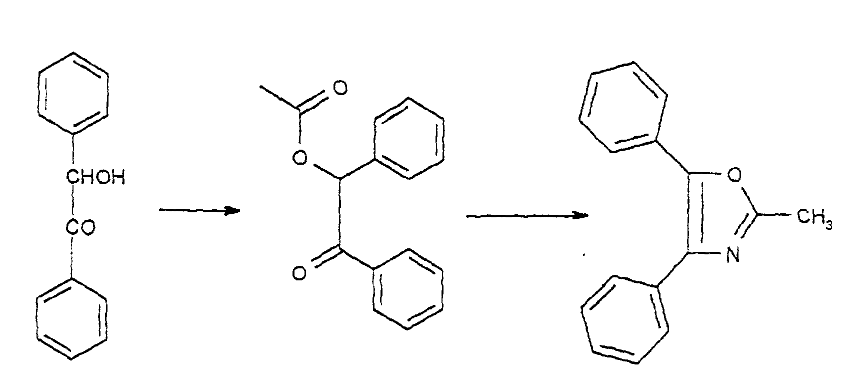

### a/ synthèse de l'acétate de 2-oxo-1,2-diphényl-éthyle

Dans un réacteur, on additionne 2 g de benzoïne avec 15 ml d'anhydride acétique et quelques gouttes d'acide sulfurique sous agitation et à température ambiante (25°C) pendant 15 heures. On ajoute de l'eau au milieu réactionnel et du dichlorométhane. La phase organique est récupérée après décantation, séchée sur sulfate de sodium, filtrée puis concentrée à sec sous vide.
On obtient 2,10 g d'une poudre blanche (rendement : 88%).

### b/ synthèse du 2-méthyl-4,5-diphényl-oxazole

Dans un réacteur, on solubilise 0,2 g de l'acétate de 2-oxo-1,2-diphényl-éthyle ci-dessus, dans 10 ml de diméthylformamide. On ajoute ensuite 0,12 g de thiourée (2éq) et on laisse réagir à reflux (160°C) pendant 10 heures. Le mélange réactionnel est extrait à l'aide de dichlorométhane, puis lavé à l'eau à 3 reprises. La phase organique est récupérée après décantation, séchée sur sulfate de sodium, filtrée puis concentrée à sec sous vide. L'huile obtenue est ensuite purifiée sur colonne de gel de silice (éluant : dichlorométhane).
On obtient 0,13 g d'une huile (rendement : 54%).
- spectre RMN¹H (200MHz ;DMSO)

| δ(ppm) | multiplicité | intégration |
|---|---|---|
| 2,5 | s | 3 |
| 7,38 | m | 6 |
| 7,53 | m | 4 |

### Exemple 3 : Préparation du [4-(2,5-diphényl-oxazole-4-yl)phényl]-diméthylamine

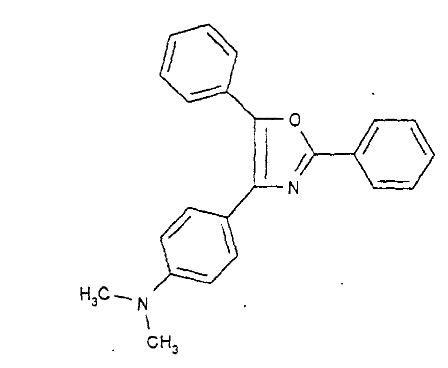

### a/ Benzoylation : préparation du benzoate de 2-(4-diméthylamino-phényl)-2-oxo-1-phényl-éthyle

Dans un réacteur, on additionne 1 g de diméthylaminobenzoïne avec 0,6 g de triéthylamine (1,5 éq) et 0,1g de diméthylaminopyridine dans 20 ml de dichlorométhane sous agitation à 0°C.
On y introduit progressivement grâce à une ampoule à brome, 0,825 g de chlorure de benzoyle en solution dans 5 ml de dichlorométhane (temps d'introduction d'une durée d'environ 10 minutes). On laisse réagir pendant 5 heures. Le milieu réactionnel est lavé à l'eau une première fois, puis à l'eau basique (soude 1N) deux fois.
La phase organique est récupérée après décantation, séchée sur sulfate de sodium, filtrée puis concentrée à sec sous vide.
On obtient 1,2 g d'une poudre légèrement jaune (rendement : 86%).

### b/ Cyclisation : synthèse du [4-(2,5-diphényl-oxazole-4-yl)phényl] diméthylamine

Dans un réacteur, on solubilise 0,5 g de benzoate de 2-(4-diméthylamino-phényl)-2-oxo-1-phényl-éthyle dans 10 ml de diméthylformamide. On ajoute ensuite 0,212 g de thiourée (2éq) et on laisse réagir à reflux (160°C) pendant 6 heures.
Le mélange réactionnel est extrait à l'aide de dichlorométhane, puis lavé à l'eau à 3 reprises. La phase organique est récupérée après décantation, séchée sur sulfate de sodium, filtrée puis concentrée à sec sous vide. Elle est ensuite purifiée sur colonne de gel de silice (éluant : dichlorométhane).
On obtient 0,12 g d'une poudre blanche (rendement : 25%).
- spectre RMN¹H(200MHz ;DMSO)

| δ(ppm) | multiplicité | Intégration |
|---|---|---|
| 2,95 | s | 6 |
| 6,77 | d | 2 |
| 7,5 | m | 8 |
| 7,7 | d | 2 |
| 8,1 | d | 2 |

### Exemple 4 : Préparation du 2-phényl-4,5-di-p-tolyl-oxazole

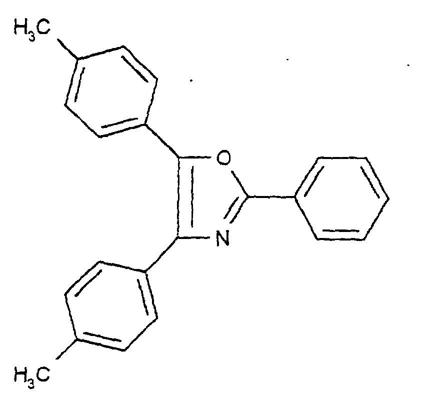

### a/ Benzoylation: synthèse du benzoate de 2-oxo-1,2-di-p-tolyl-éthyle

Dans un réacteur, on additionne 1 g de diméthylbenzoïne avec 0,63 g de triéthylamine (1,5 éq) et 0,1 g de diméthylaminopyridine, dans 20 ml de dichlorométhane, sous agitation et à 0°C.
On introduit progressivement grâce à une ampoule à brome, 0,88 g de chlorure de benzoyle en solution dans 5 ml de dichlorométhane (temps d'introduction d'une durée d'environ 10 minutes). On laisse réagir pendant 5 heures. Le milieu réactionnel est alors lavé à l'eau trois fois. La phase organique est récupérée après décantation, séchée sur sulfate de sodium, filtrée puis concentrée à sec sous vide. On obtient 1,2 g d'une poudre blanche (rendement : 84%).

### b/ Cyclisation : synthèse du 2-phényl-4,5-di-p-tolyl-oxazole

Dans un réacteur, on solubilise 0,5 g de benzoate de 2-oxo-1,2-di-p-tolyl-éthyle dans 10 ml de diméthylformamide. On ajoute ensuite 0,22 g de thiourée (2éq) et on laisse réagir à reflux (160°C) pendant 5 heures. On verse la solution sur un mélange eau/glace. Le produit précipité est lavé à l'eau, filtré sur fritté sous vide, placé une nuit au dessiccateur avant d'être purifié sur colonne de gel de silice avec du dichlorométhane comme éluant.

On obtient 0,27 g d'une poudre beige (rendement : 60%).
- spectre RMN¹H (200MHz; CDCl₃)

| δ(ppm) | Multiplicité | intégration |
|---|---|---|
| 2,3 | s | 6 |
| 7,1 | m | 4 |
| 7,5 | m | 7 |
| 8,1 | d | 2 |

### Exemple 5 : Préparation du 4-[4-(4-fluoro-phenyl)-2-phenyloxazole-5-yl]-pyridine

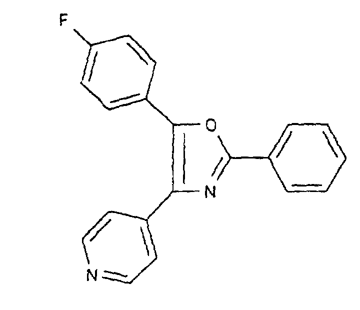

Dans un réacteur, on solubilise 0,72 g de benzoate de 1-(4-fluoro-phényl)-2-oxo-2-pyridine-4-yl-éthyle (préparé selon J.O.C., *1984*, 27, 72) dans 10 ml de diméthylformamide. On ajoute ensuite 0,32 g de thiourée (2éq) et on laisse réagir à reflux (160°C) pendant 6 heures.
Le mélange réactionnel est extrait à l'aide de dichlorométhane puis lavé 2 fois à l'eau, séché sur sulfate de sodium, filtré et enfin mis à sec sous vide.
Le produit obtenu est alors purifié sur colonne de silice (éluant : dichlorométhane/méthanol : 99,5/0,5).
On obtient 0,18 g d'une poudre orange (rendement : 27%).
- spectre RMN¹H (200MHz; DMSO)

| δ(ppm) | multiplicité | intégration |
|---|---|---|
| 7,35 | m | 2 |
| 7,6 | m | 5 |
| 7,72 | m | 2 |
| 8,15 | m | 2 |
| 8,64 | m | 2 |

### Exemple 6 : Préparation du 2-(3-benzyloxy-propyl)-4,5-diphenyl-oxazole

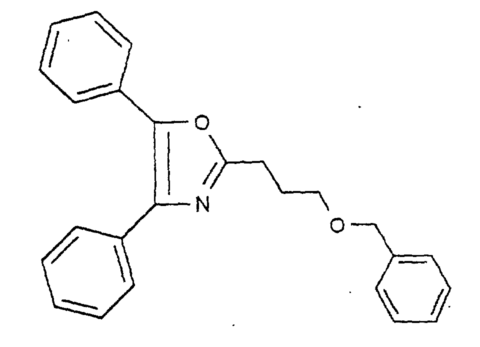

### a/ synthèse du 4-benzyloxy-butanoate de 2-oxo-1,2-diphényl-éthyle

Dans un réacteur sous atmosphère d'azote, on solubilise 1,5 g d'acide 4-benzyloxybutanoïque dans 10 ml de toluène anhydre. On ajoute ensuite 1,45 ml de chlorure d'oxalyle à 98% (2,1éq) et 4 gouttes de diméthyformamide afin de catalyser la réaction. On laisse agiter à température ambiante pendant 1 heure. La solution est concentrée à sec sous vide. L'huile orange obtenue est solubilisée dans le dichlorométhane puis introduite goutte à goutte dans un réacteur placé sous atmosphère d'azote, contenant une solution de 1,18 g (0,7éq) de benzoïne et 1,58 g (2 éq) de triéthylamine dans du dichorométhane. On note un dégagement immédiat. On laisse le milieu réactionnel sous agitation pendant 3 heures à température ambiante. Le mélange réactionnel est lavé 3 fois à l'eau, séché sur sulfate de sodium anhydre, filtré et enfin mis à sec. Le produit estérifié est alors isolé sur colonne de silice (éluant : dichlorométhane).
On obtient 0,3 g d'une huile jaune (rendement : 60%).

### b/ Cyclisation : synthèse du 2-(3-benzyloxy-propyl)-4,5-diphényl-oxazole

Dans un réacteur, on solubilise 0,3 g de 4-benzyloxy-butanoate de 2-oxo-1,2-diphényl-éthyle dans 10 ml de diméthylformamide. On ajoute ensuite 0,12 g de thiourée (2éq) et on laisse réagir à reflux (160°C) pendant 5 heures. Le mélange réactionnel est extrait à l'aide de dichlorométhane puis lavé 3 fois à l'eau, séché sur sulfate de sodium, filtré et enfin mis à sec sous vide.
Le produit obtenu est alors purifié sur colonne de silice (éluant : dichlorométhane).
On obtient 0,1 g d'une huile jaune (rendement : 35%).
- spectre RMN¹H (200MHz ;DMSO)

| δ(ppm) | Multiplicité | intégration |
|---|---|---|
| 2,05 | M | 2 |
| 2,9 | T | 2 |
| 3,55 | T | 2 |
| 4,47 | S | 2 |
| 7,3 à 7,6 | m | 15 |

### Exemple 7 : Préparation du 2-phényl-4-terbutyl-5-éthyl oxazole

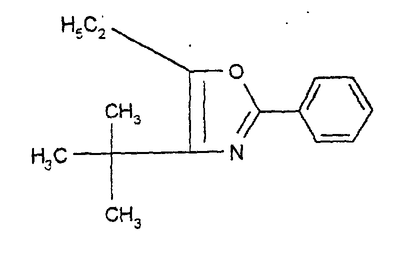

### a/ Benzoylation : synthèse du benzoate de 1-éthyl-3,3-diméthyl-2-oxo-butyle

Dans un réacteur, on additionne 0,31 g de 4-hydroxy-2-2-diméthyl-hexane-3-one (préparé selon J.O.C., *1991,* 56, 3118) avec 0,3 g de triéthylamine (1,5 éq) et quelques mg de diméthylaminopyridine (catalyseur), le tout dans 5 ml de dichlorométhane sous agitation et à 0°C.
On introduit progressivement grâce à une ampoule à brome, 0,42 g de chlorure de benzoyle (1,5 éq) en solution dans 5 ml de dichlorométhane (temps d'introduction d'une durée d'environ 10 minutes). On laisse sous agitation pendant 3 heures à température ambiante.
Le mélange réactionnel est lavé 2 fois à l'eau, puis 2 fois à l'eau saturée en bicarbonate de sodium. On sèche sur sulfate de sodium anhydre, on filtre et enfin on met à sec sous vide. Le produit benzoylé est alors isolé sur colonne de silice (éluant : dichlorométhane).
On obtient 0,31 g d'une huile jaune (rendement : 60%).

### b/ Cyclisation : synthèse du 2-phényl-4-terbutyl-5-éthyl oxazole

Dans un réacteur, on solubilise 0,31 g de benzoate de 1-éthyl-3,3-diméthyl-2-oxo-butyle dans 8 ml de diméthylformamide. On ajoute ensuite 0,75 g de thiourée (5éq) et on laisse réagir à reflux (160°C) pendant 6 heures. Le mélange réactionnel est extrait à l'aide de dichlorométhane puis lavé 3 fois à l'eau, séché sur sulfate de sodium anhydre, filtré et enfin mis à sec sous vide.
Le produit obtenu est alors purifié sur colonne de silice (éluant : dichlorométhane).

On obtient 0,1 g d'une huile jaune (rendement : 35%).
- spectre RMN¹H (200MHz ;DMSO)

| δ(ppm) | multiplicité | Intégration |
|---|---|---|
| 1,25 | t | 3 |
| 1,35 | s | 9 |
| 2,8 | q | 2 |
| 7,4 | m | 3 |
| 8 | m | 2 |

### Exemple 8 : Préparation du 2-5 diphényl-4-terbutyl-oxazole

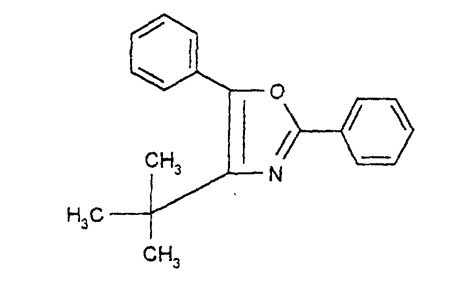

### a/ Benzoylation : synthèse du benzoate de 3,3-diméthyl-2-oxo-1-phényl-butyle

Dans un réacteur, on additionne 0,45 g de 1-hydroxy-3,3-diméthyl-1-phényl-butan-2-one (préparé selon J.O.C., *1991*, 56, 3118) avec 0,3 g de triéthylamine (1,5 éq) et quelques mg de diméthylaminopyridine, le tout dans 5 ml de dichlorométhane sous agitation et à 0°C.
On introduit progressivement grâce à une ampoule à brome, 0,42 g de chlorure de benzoyle (1,5 éq) en solution dans 5 ml de dichlorométhane (temps d'introduction d'une durée d'environ 10 minutes). On laisse sous agitation pendant 3 heures à température ambiante. Le mélange réactionnel est lavé 2 fois à l'eau puis 2 fois à l'eau saturée en bicarbonate de sodium. Il est ensuite séché sur sulfate de sodium anhydre, filtré et enfin mis à sec sous vide.
Le produit benzoylé est alors isolé sur colonne de silice (éluant : dichlorométhane).
On obtient 0,40 g d'une huile jaune (rendement : 60%).

### b/ cyclisation : synthèse du 2-5 diphényl-4-terbutyl-oxazole

Dans un réacteur, on solubilise 0,15 g de benzoate de 3,3-diméthyl-2-oxo-1-phényl-butyle dans 8 ml de diméthylformamide. On ajoute ensuite 0,2 g de thiourée (5éq) et on laisse réagir à reflux (160°C) pendant 15 heures.
Le mélange réactionnel est extrait à l'aide de dichlorométhane puis lavé 3 fois à l'eau, séché sur sulfate de sodium anhydre, filtré et enfin mis à sec sous vide.
Le produit obtenu est alors purifié sur colonne de silice (éluant : dichlorométhane).
On obtient 0,06 g d'une huile jaune (rendement : 20%).
- spectre de masse : ( MH )⁺ = M/z = 278

## Revendications

1. Procédé de préparation d'oxazoles trisubstitués de formule (II) : 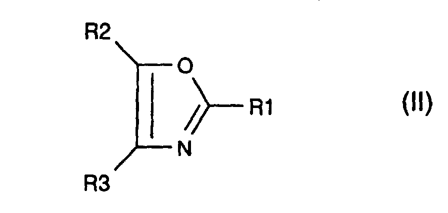 par réaction de thiourée sur la dicétone-ester correspondante de formule (I) : 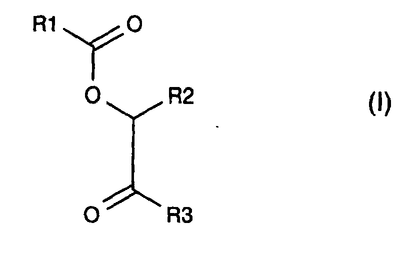 dans lesquelles les radicaux R₁, R₂ et R₃, identiques ou différents, sont choisis parmi:
- les radicaux hydrocarbonés linéaires ou ramifiés, saturés ou insaturés, en C₁-C₁₂, éventuellement substitués par -OR, -SR, -NRR', -COOR, -CN, -SOₙCH₃, -CF₃ et/ou halogène, avec R et R', identiques ou différents, représentant l'hydrogène, un radical hydrocarboné linéaire, ramifié ou cyclique, saturé ou insaturé en C₁-C₁₂ et n = 0, 1 ou 2;
- les radicaux aryles éventuellement substitués par un radical hydrocarboné linéaire ou ramifié, saturé ou insaturé, en C₁-C₁₂, un radical -OR, -NRR', -COOR, -CN, -SOₙCH₃, -SR, -CF₃ et/ou halogène, R, R' et n ayant les mêmes significations que ci-dessus, et/ou
- les radicaux hétérocycliques en C₅₋₁₀, saturés ou insaturés, comportant au moins un hétéroatome choisi parmi N, S et/ou O, éventuellement substitués par un radical hydrocarboné linéaire ou ramifié en C₁-C₁₂, un radical -OR, -NRR', -COOR, -CN, -SOₙCH₃, -SR, -CF₃ et/ou halogène, R, R' et n ayant les mêmes significations que ci-dessus,
la réaction étant effectuée dans le DMF (diméthylformamide).

2. Procédé selon la revendication 1, dans lequel les radicaux R₁, R₂ et R₃, identiques ou différents, sont choisis parmi :
- les radicaux hydrocarbonés saturés, linéaires ou ramifiés, en C₁-C₆,
- les radicaux hydrocarbonés linéaires ou ramifiés, saturés, en C₂-C₆ substitués par -OR avec R représentant un radical phényl ou alkylaryl;
- le radical phényle;
- les radicaux phényles substitués par un radical hydrocarboné saturé en C₁-C₄,
- les radicaux phényles substitués par un radical -NRR' avec R et R' représentant un radical hydrocarboné saturé en C₁-C₄,
- les radicaux phényles substitués par au moins un halogène,
- les radicaux hétérocycliques en C₅₋₆, insaturés, comportant au moins un atome d'azote dans le cycle.

3. Procédé selon l'une des revendications précédentes, dans lequel les radicaux R₁, R₂ et R₃, identiques ou différents, sont choisis parmi :
- les radicaux méthyle, éthyle, propyle, n-butyle, iso-butyle ou ter-butyle;
- les radicaux hydrocarbonés linéaires ou ramifiés, saturés, en C₂-C₆ substitués par -OR avec R représentant un radical -(CH₂)ₙ-phényl avec n = 1 à 4;
- les radicaux phényles substitués par un radical méthyle, éthyle, propyle, n-butyle, iso-butyle ou ter-butyle;
- les radicaux phényles substitués par un radical -NRR' avec R et R' représentant un radical méthyle, éthyle, propyle, n-butyle, iso-butyle ou ter-butyle;
- les radicaux phényles substitués par au moins un fluor.

4. Procédé selon l'une des revendications précédentes, dans lequel la réaction est effectuée à une température d'au moins 130°C.

5. Procédé selon l'une des revendications précédentes, dans lequel la réaction est effectuée à une température supérieure à 140°C.

6. Procédé selon l'une des revendications précédentes, dans lequel la réaction est effectuée à reflux du solvant.

7. Procédé selon l'une des revendications précédentes comportant les étapes suivantes :
- on solubilise la dicétone-ester (I) et la thiourée dans le DMF (diméthylformamide), de préférence à raison de environ 2 équivalents de thiourée pour 1 équivalent de dicétone-ester;
- on chauffe le mélange à une température d'au moins 130°C, pendant le temps nécessaire à l'accomplissement de la totalité de la réaction, et qui peut être de l'ordre de 5 à 10 heures.

8. Procédé selon la revendication 7, dans lequel le produit recherché est isolé selon les méthodes usuelles, telles que précipitation, filtration, extraction.

9. Procédé selon l'une des revendications 7 à 8, dans lequel le produit recherché est lavé et/ou séché et/ou recristallisé et/ou purifié selon les méthodes usuelles.

## Patentansprüche

1. Verfahren zur Herstellung von dreifach substituierten Oxazolen der Formel (II): 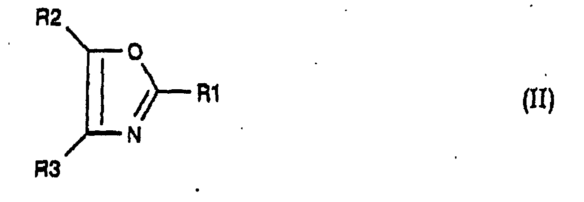 durch Umsetzung von Thioharnstoff mit dem korrespondierenden Diketonester der Formel (I): 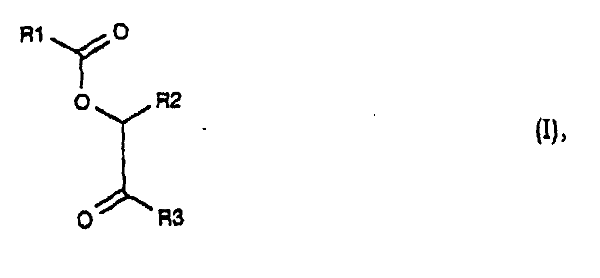 wobei die Gruppen R₁, R₂ und R₃, die gleich oder verschieden sind, unter den folgenden Gruppen ausgewählt sind:
- den geradkettigen oder verzweigten, gesättigten oder ungesättigten Kohlenwasserstoffgruppen mit 1 bis 12 Kohlenstoffatomen, die gegebenenfalls mit -OR, -SR, -NRR', -COOR, -CN, -SOₙCH₃, -CF₃ und/oder Halogen substituiert sind, wobei R und R', die gleich oder verschieden sind, Wasserstoff, eine geradkettige, verzweigte oder cyclische, gesättigte oder ungesättigte Kohlenwasserstoffgruppe mit 1 bis 12 Kohlenstoffatomen bedeutet und n = 0, 1 oder 2;
- den Arylgruppen, die gegebenenfalls mit einer geradkettigen oder verzweigten, gesättigten oder ungesättigten Kohlenwasserstoffgruppe mit 1 bis 12 Kohlenstoffatomen, -OR, -NRR', -COOR, -CN, -SOₙCH₃, -SR, -CF₃ und/oder Halogen substituiert sind, wobei R, R' und n die oben angegebenen Bedeutungen aufweisen, und/oder
- den heterocyclischen Gruppen mit 5 bis 10 Kohlenstoffatomen, die gesättigt oder ungesättigt sind, mindestens ein Heteroatom enthalten, das unter N, S und/oder O ausgewählt ist, und gegebenenfalls mit einer geradkettigen oder verzweigten C₁₋₁₂-Kohlenwasserstoffgruppe, -OR, -NRR', -COOR, -CN, -SOₙCH₃, -SR, -CF₃ und/oder Halogen substituiert sind, wobei R, R' und n die oben angegebenen Bedeutungen aufweisen,
wobei die Umsetzung in DMF (Dimethylformamid) durchgeführt wird.

2. Verfahren nach Anspruch 1, wobei die Gruppen R₁, R₂ und R₃, die identisch oder verschieden sind, unter den folgenden Gruppen ausgewählt sind:
- gesättigten, geradkettigen oder verzweigten Kohlenwasserstoffgruppen mit 1 bis 6 Kohlenstoffatomen;
- geradkettigen oder verzweigten, gesättigten Kohlenwasserstoffgruppen mit 2 bis 6 Kohlenstoffatomen, die mit -OR substituiert sind, wobei R eine Phenyl- oder Alkylarylgruppe bedeutet;
- Phenyl;
- Phenylgruppen, die mit einer gesättigten Kohlenwasserstoffgruppe mit 1 bis 4 Kohlenstoffatomen substituiert sind;
- Phenylgruppen, die mit einer Gruppe -NRR' substituiert sind, wobei R und R' eine gesättigte Kohlenwasserstoffgruppe mit 1 bis 4 Kohlenstoffatomen bedeuten;
- Phenylgruppen, die mit mindestens einem Halogen substituiert sind, und
- ungesättigten heterocyclischen Gruppen mit 5 oder 6 Kohlenstoffatomen, die mindesten ein Stickstoffatom im Ring enthalten.

3. Verfahren nach einem der vorhergehenden Ansprüche, wobei die Gruppen R₁, R₂ und R₃, die identisch oder voneinander verschieden sind, unter den folgenden Gruppen ausgewählt sind:
- den Gruppen Methyl, Ethyl, Propyl, n-Butyl, Isobutyl oder *t*-Butyl;
- den geradkettigen oder verzweigten, gesättigten Kohlenwasserstoffgruppen mit 2 bis 6 Kohlenstoffatomen, die mit -OR substituiert sind, wobei R eine Gruppe -(CH₂)ₙ-Phenyl mit n = 1 bis 4 bedeutet;
- den Phenylgruppen, die mit Methyl, Ethyl, Propyl, n-Butyl, Isobutyl oder *t*-Butyl substituiert sind;
- den Phenylgruppen, die mit einer Gruppe -NRR' substituiert sind, wobei R und R' Methyl, Ethyl, Propyl, n-Butyl, Isobutyl oder *t*-Butyl bedeuten;
- den Phenylgruppen, die mit mindestens einem Fluoratom substituiert sind.

4. Verfahren nach einem der vorhergehenden Ansprüche, wobei die Umsetzung bei einer Temperatur von mindestens 130 °C durchgeführt wird.

5. Verfahren nach einem der vorhergehenden Ansprüche, wobei die Umsetzung bei einer Temperatur über 140 °C durchgeführt wird.

6. Verfahren nach einem der vorhergehenden Ansprüche, wobei die Umsetzung bei der Rückflusstemperatur des Lösungsmittels durchgeführt wird.

7. Verfahren nach einem der vorhergehenden Ansprüche mit den folgenden Schritten:
- der Diketonester (I) und der Thioharnstoff werden in DMF (Dimethylformamid) vorzugsweise in einer Menge von etwa 2 Äquivalenten Thioharnstoff auf 1 Äquivalent Diketonester solubilisiert; und
- das Gemisch wird auf eine Temperatur von mindestens 130 °C während einer Zeitspanne erwärmt, die erforderlich ist, damit die Umsetzung vollständig ablaufen kann, wobei diese Zeitspanne in der Größenordnung von 5 bis 10 h liegen kann.

8. Verfahren nach Anspruch 7, wobei das gewünschte Produkt nach herkömmlichen Verfahren, wie Fällen, Filtrieren und Extrahieren, isoliert wird.

9. Verfahren nach einem der Ansprüche 7 bis 8, wobei das gewünschte Produkt nach herkömmlichen Verfahren gewaschen und/oder getrocknet und/oder umkristallisiert und/oder gereinigt wird.

## Claims

1. Process for the preparation of trisubstituted oxazoles of formula (II): 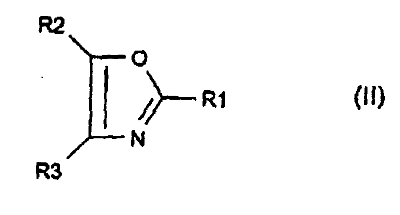 by reaction of thiourea with the corresponding diketone ester of formula (I): 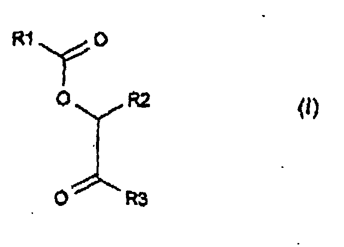 in which the R₁, R₂ and R₃ radicals, which are identical or different, are chosen from:
- saturated or unsaturated and linear or branched C₁-C₁₂ hydrocarbonaceous radicals optionally substituted by -OR, -SR, -NRR', -COOR, -CN, -SOₙCH₃, -CF₃ and/or halogen, with R and R', which are identical or different, representing hydrogen or a saturated or unsaturated and linear, branched or cyclic C₁-C₁₂ hydrocarbonaceous radical arid n = 0, 1 or 2;
- aryl radicals optionally substituted by a saturated or unsaturated and linear or branched C₁-C₁₂ hydrocarbonaceous radical or an -OR, -NRR', -COOR, -CN, -SOₙCH₃, -SR, -CF₃ and/or halogen radical, R, R' and n having the same meanings as above, and/or
- saturated or unsaturated C₅₋₁₀ heterocyclic radicals comprising at least one heteroatom chosen from N, S and/or O which are optionally substituted by a linear or branched C₁-C₁₂ hydrocarbonaceous radical or an -OR, -NRR', -COOR, -CN, -SOₙCH₃, -SR, -CF₃ and/or halogen radical, R, R' and n having the same meanings as above, the reaction being carried out in DMF (dimethylformamide).

2. Process according to Claim 1, in which the R₁, R₂ and R₃ radicals, which are identical or different, are chosen from:
- saturated, linear or branched, C₁-C₆ hydrocarbonaceous radicals,
- saturated, linear or branched, C₂-C₆ hydrocarbonaceous radicals substituted by -OR with R representing a phenyl or alkylaryl radical;
- the phenyl radical;
- phenyl radicals substituted by a saturated C₁-C₄ hydrocarbonaceous radical,
- phenyl radicals substituted by an -NRR' radical with R and R' representing a saturated C₁-C₄ hydrocarbonaceous radical,
- phenyl radicals substituted by at least one halogen,
- unsaturated C₅₋₆ heterocyclic radicals comprising at least one nitrogen atom in the ring.

3. Process according to either of the preceding claims, in which the R₁, R₂ and R₃ radicals, which are identical or different, are chosen from:
- methyl, ethyl, propyl, n-butyl, isobutyl or tert-butyl radicals;
- saturated, linear or branched, C₂-C₆ hydrocarbonaceous radicals substituted by -OR with R representing a -(CH₂)ₙ- phenyl radical with n = 1 to 4;
- phenyl radicals substituted by a methyl, ethyl, propyl, n-butyl, isobutyl or tert-butyl radical;
- phenyl radicals substituted by an -NRR' radical with R and R' representing a methyl, ethyl, propyl, n-butyl, isobutyl or tert-butyl radical;
- phenyl radicals substituted by at least one fluorine.

4. Process according to one of the preceding claims, in which the reaction is carried out at a temperature of at least 130°C.

5. Process according to one of the preceding claims, in which the reaction is carried out at a temperature of greater than 140°C.

6. Process according to one of the preceding claims, in which the reaction is carried out at reflux of the solvent.

7. Process according to one of the preceding claims, comprising the following stages:
- the diketone ester (I) and thiourea are dissolved in DMF (dimethylformamide), preferably in a proportion of approximately 2 equivalents of thiourea per 1 equivalent of diketone ester; and
- the mixture is heated at a temperature of at least 130°C for the time needed to bring the reaction to completion, which can be of the order of 5 to 10 hours.

8. Process according to Claim 7, in which the desired product is isolated according to the usual methods, such as precipitation, filtration or extraction.

9. Process according to either of Claims 7 and 8, in which the desired product is washed and/or dried and/or recrystallized and/or purified according to the usual methods.
